# EUROPÄISCHE PATENTSCHRIFT

(11) **EP 0 736 294 B1**
(45) Veröffentlichungstag und Bekanntmachung des Hinweises auf die Patenterteilung: **11.10.2000**
(21) Anmeldenummer: 96102220.9
(22) Anmeldetag: 15.02.1996
(51) Int. Cl.: A61F 2/60, A61F 2/76

(54) **Unterschenkel-Beinprothese**
Leg prosthesis
Prothèse de jambe

(30) Priorität: 07.03.1995 DE 19507894
(43) Veröffentlichungstag der Anmeldung: 09.10.1996
(73) Patentinhaber: Otto Bock Orthopädische Industrie Besitz- und Verwaltungs-Kommanditgesellschaft, 37115 Duderstadt (DE)
(72) Erfinder: Becker, Karl, D-37115 Duderstadt (DE); Hiemisch, Christian, D-37115 Duderstadt (DE); Schaarschuch, Roland, S-36043 Aryd (SE); Zenner, Harry, NL-5684 ZC Best (NL)
(74) Vertreter: Gramm, Werner, Prof. Dipl.-Ing.

(56) Entgegenhaltungen:
- WO-A-93/17640
- GB-A- 2 274 398

## Beschreibung

Die Erfindung betrifft eine Unterschenkel-Beinprothese mit einem den Unterschenkelstumpf aufnehmenden Unterschenkeltrichter und einer Rohrverlängerung, deren oberes Ende mit dem Unterschenkeltrichter über einen am unteren Ende des Unterschenkeltrichters befestigten Adapter und deren unteres Ende mit einem Fußpaßteil verbunden sind, wobei der Adapter gegenüber dem unteren Trichterende auf einer Kugelsegmentfläche verschieb- und festlegbar ist, deren durch zwei orthogonale Schnittebenen gebildeten Kreisbogen in der Sagital- und Frontalebene liegen, wobei die Kugelsegmentfläche die einzige Justierfläche für den Adapter darstellt.

Eine derartige Ausführungsform läßt sich der GB-A-2 274 398 entnehmen. Vergleichbar erscheint auch eine Ausführungsform gemäß der US-A-3,422,462.

Der Erfindung liegt die Aufgabe zugrunde, die eingangs beschriebene Unterschenkel-Beinprothese hinsichtlich ihrer Anpassung an den Unterschenkelstumpf zu vereinfachen.

In Verbindung mit den eingangs beschriebenen Merkmalen wird diese Aufgabe gemäß der Erfindung dadurch gelöst, daß der Mittelpunkt der die Kugelsegmentfläche bildenden Kugel die Kniemitte ist.

Während bei der Ausführungsform gemäß der GB-A-2 274 398 der Mittelpunkt der die Kugelsegmentfläche bildenden Kugel beträchtlich unterhalb der Kniemitte liegt, läßt sich durch die Verlagerung des genannten Kugel-Mittelpunktes in die Kniemitte durch einfache Adapterverstellung einfach und schnell eine Korrektur im Sinne der Beugung und Streckung sowie der An- und Abspreizung durchführen.

Auch bei einer Veränderung der Position unterhalb des Schaftes im Sinne Flexion, Extension, Abduktion und Adduktion wird das Verhältnis in die Mitte zur Position des Prothesenfusses niemals verändert. Somit können auch weniger qualifizierte Techniker Positionsveränderungen vornehmen, ohne schwerwiegende Aufbaufehler zu erzeugen. Justiert wird ausschließlich der Unterschenkelschaft gegenüber der Normalstellung, wenn durch außergewöhnliche Stumpfposition ein Korrekturbedarf vorliegt. Eine Nachjustierung im Prothesenunterbau muß also nicht vorgenommen werden.

Um für verschiedene Prothesengrößen nur wenige Bauteile vorhalten zu müssen, kann ein kompletter Bausatz zur Herstellung von Unterschenkel-Beinprothesen z.B. nur drei Unterschenkeltrichter unterschiedlicher Größe umfassen, die jeweils so ausgelegt sind, daß der in dem jeweiligen Größenbereich längst denkbare Stumpf hineinpaßt. Die in den drei Größenbereichen noch vorhandenen unterschiedlichen Stumpflängen lassen sich dann durch Einlage von einem oder mehreren Stützkissen in dem Unterschenkeltrichter ausgleichen. Ein unterschiedlicher Stumpumfang wird durch Nachspannen des schalenförmigen Unterschenkeltrichters mit Hilfe einer Spannvorrichtung ausgeglichen. Die unterschiedliche Beinlänge läßt sich in einfacher Weise vor Ort durch Ablängen des im Bausatz befindlichen Schaftrohres anpassen.

Vorzugsweise ist noch ein kosmetisches Knöchelformteil vorgesehen, das mit seinem oberen Ende das untere Ende des Unterschenkeltrichters gleitend übergreift und an seinem unteren Ende eine ringförmige Dichtlippe aufweist zum spritzwasserdichten Anschluß an ein Fußpaßteil.

In der Zeichnung ist eine als Beispiel dienende Ausführungsform der Erfindung dargestellt. Es zeigen:

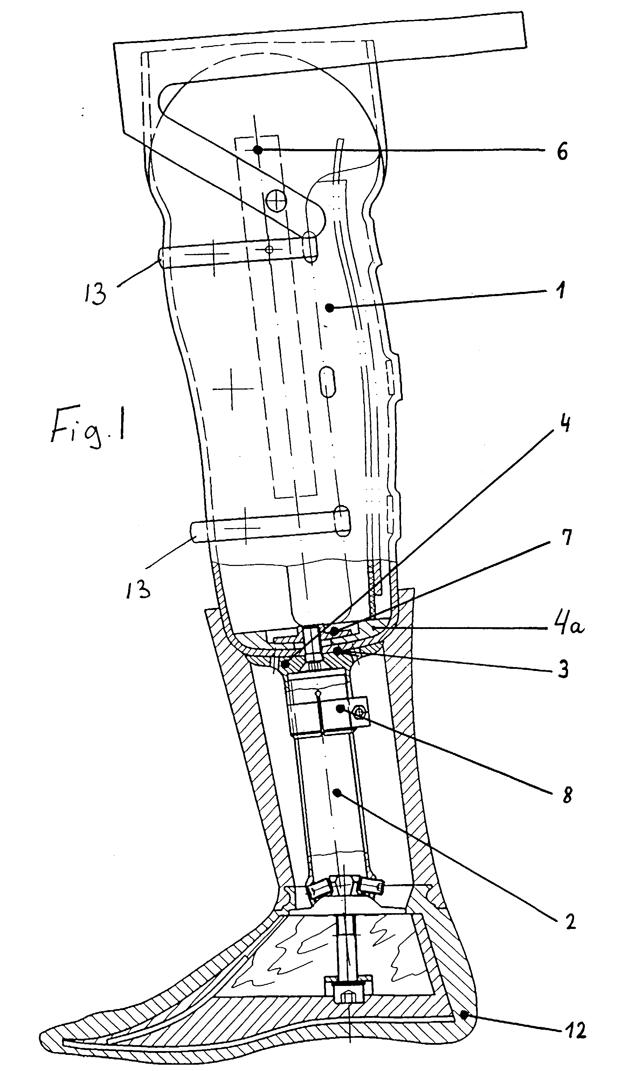

- **Figur 1 -**: eine Unterschenkel-Beinprothese in Seitenansicht und zum Teil im Längsschnitt und

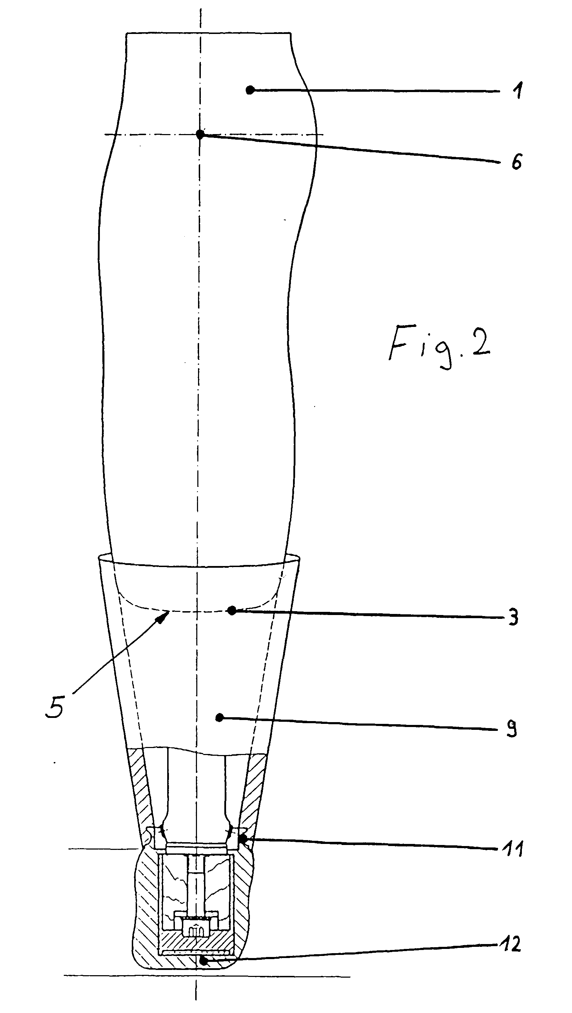

- **Figur 2 -**: die Darstellung gemäß Figur 1 in Rückansicht.

Dargestellt ist eine Unterschenkel-Beinprothese mit einem den Unterschenkelstumpf aufnehmenden Unterschenkeltrichter 1 und einer Rohrverlängerung 2, die über einen am unteren Ende 3 des Unterschenkeltrichters 1 befestigten Adapter 4 miteinander verbunden sind.

Der Adapter 4 ist gegenüber dem unteren Trichterende 3 auf einer Kugelsegmentfläche 5 verschieb- und festlegbar, deren durch zwei orthogonale Schnittebenen gebildeten Kreisbogen in der Sagittal- und Frontebene liegen; der Mittelpunkt 6 der die Kugelsegmentfläche 5 bildenden Kugel ist die Kniemitte.

Dem Adapter 4 ist innerhalb des unteren Trichterendes 3 eine entsprechend geformte Adapterschale 4a zugeordnet, die von oben durch eine Spannscheibe 7 beaufschlagt wird.

Die Rohrverlängerung 2 ist am Adapter 4 über eine Spannschelle 8 drehjustierbar befestigt.

Vorgesehen ist ferner ein kosmetisches Knöchelformteil 9, das mit seinem oberen Ende das untere Ende 3 des Unterschenkeltrichters 1 gleitend übergreift und an seinem unteren Ende eine ringförmige Dichtlippe 11 aufweist zum spritzwasserdichten Anschluß an ein Fußpaßteil 12. Letzteres ist an dem unteren Ende der Rohrverlängerung 2 über einen pyramidenförmig gestalteten Adapter 10 lösbar befestigt.

Ein unterschiedlicher Stumpfumfang wird durch Nachspannen des schalenförmig ausgebildeten Unterschenkeltrichters 1 mit Hilfe einer Spannvorrichtung 13 ausgeglichen.

## Patentansprüche

1. Unterschenkel-Beinprothese mit einem den Unterschenkelstumpf aufnehmenden Unterschenkeltrichter (1) und einer Rohrverlängerung (2), deren oberes Ende mit dem Unterschenkeltrichter (1) über einen am unteren Ende (3) des Unterschenkeltrichters (1) befestigten Adapter (4) und deren unteres Ende mit einem Fußpaßteil (12) verbunden sind, wobei der Adapter (4) gegenüber dem unteren Trichterende (3) auf einer Kugelsegmentfläche (5) verschieb- und festlegbar ist, deren durch zwei orthogonale Schnittebenen gebildeten Kreisbogen in der Sagittal- und Frontalebene liegen, wobei die Kugelsegmentfläche (5) die einzige Justierfläche für den Adapter (4) darstellt, **dadurch gekennzeichnet,** daß der Mittelpunkt (6) der die Kugelsegmentfläche (5) bildenden Kugel die Kniemitte ist.

2. Unterschenkel-Beinprothese nach Anspruch 1, **dadurch gekennzeichnet, daß** dem Adapter (4) innerhalb des unteren Trichterendes (3) eine entsprechend geformte Adapterschale (4a) zugeordnet ist, die von oben durch eine Spannscheibe (7) beaufschlagt wird.

3. Unterschenkel-Beinprothese nach Anspruch 1 oder 2, **dadurch gekennzeichnet, daß** die Rohrverlängerung (2) am Adapter (4) über eine Spannschelle (8) drehjustierbar befestigt ist.

4. Unterschenkel-Beinprothese nach Anspruch 1, 2 oder 3, **gekennzeichnet durch** ein kosmetisches Knöchelformteil (9), das mit seinem oberen Ende (3) das untere Ende des Unterschenkeltrichters (1) gleitend übergreift und an seinem unteren Ende eine ringförmige Dichtlippe (11) aufweist zum spritzwasserdichten Anschluß an das Fußpaßteil (12).

## Claims

1. A lower leg prosthesis with a lower leg funnel (1) receiving the lower leg stump and a tube extension (2), whose upper end is connected to the lower leg funnel (1) via an adapter (4) secured to the lower end (3) of the lower leg funnel (1) and whose lower end is connected to a matching foot part (12), the adapter (4) being displaceable and lockable relative to the lower funnel end (3) on a spherical segment surface (5), whose arcs formed by two orthogonal sectional planes lie in the sagittal and front plane, the spherical segment surface (5) representing the single plane of adjustment for the adapter (4),
characterised in that the centre point (6) of the sphere forming the spherical segment surface (5) is the centre of the knee.

2. A lower leg prosthesis according to claim 1,
characterised in that a correspondingly shaped adapter casing (4a), which is acted upon from above by a tensioning disk (7), is associated with the adapter (4) within the lower funnel end (3).

3. A lower leg prosthesis according to claim 1 or 2,
characterised in that the tube extension (2) is secured to the adapter (4) via a clamping ring (8) so as to be rotatably adjustable.

4. A lower leg prosthesis according to claim 1, 2 or 3,
characterised in that a cosmetic moulded ankle element (9), which engages in sliding fashion with its upper end (3) over the lower end of the lower leg funnel (1) and at its lower end comprises an annular sealing lip (11) for connection to a matching foot part (12) in a splash-proof manner.

## Revendications

1. Prothèse de la jambe inférieure comprenant un entonnoir de jambe inférieure (1), recevant le moignon de la jambe inférieure, et une rallonge tubulaire (2) dont l'extrémité supérieure est reliée avec l'entonnoir de jambe inférieure (1) par un adaptateur (4) fixé à l'extrémité inférieure (3) de l'entonnoir de jambe inférieure (1), et dont l'extrémité inférieure est reliée à une pièce d'adaptation du pied (12), l'adaptateur (4) pouvant être déplacé et immobilisé par rapport à l'extrémité inférieure (3) de l'entonnoir sur un surface en forme de segment sphérique, dont les arcs de cercle formés par deux plans de coupe orthogonaux s'étendent dans le plan sagittal et frontal, la surface en forme de segment sphérique (5) représentant la seule surface de réglage pour l'adaptateur (4), caractérisée en ce que le centre (6) de la sphère constituant la surface en forme de segment sphérique (5) est le centre du genou.

2. Prothèse de la jambe inférieure selon la revendication 1, caractérisée en ce qu'à l'intérieur de l'extrémité inférieure (3) de l'entonnoir, une coupelle d'adaptateur (4a) est associée à l'adaptateur (4) et formée de manière correspondante, cette coupelle étant mise sous contrainte d'en haut par une rondelle de serrage (7).

3. Prothèse de la jambe inférieure selon la revendication 1 ou 2, caractérisée en ce que la rallonge tubulaire (2) est fixée à l'adaptateur (4) de manière réglable angulairement par l'intermédiaire d'un collier de serrage (8).

4. Prothèse de la jambe inférieure selon la revendication 1, 2 ou 3, caractérisée par une pièce de forme cosmétique (9) pour la région de la cheville, qui par son extrémité supérieure chevauche de façon glissante l'extrémité inférieure (3) de l'entonnoir de jambe inférieure (1), et présente à son extrémité inférieure une lèvre d'étanchéité annulaire (11) pour se raccorder à la pièce d'adaptation du pied (12) de manière étanche aux projections d'eau.
